Europäisches Patentamt

European Patent Office

Office européen des brevets

(11) Veröffentlichungsnummer: **0 086 866**
**A1**

## (12) EUROPÄISCHE PATENTANMELDUNG

(21) Anmeldenummer: 82106182.7

(22) Anmeldetag: 10.07.82

(51) Int. Cl.³: **G 01 N 33/08**

(30) Priorität: 17.02.82 DE 8204403 U

(43) Veröffentlichungstag der Anmeldung:
31.08.83 Patentblatt 83/35

(84) Benannte Vertragsstaaten:
AT BE CH DE FR GB IT LI LU NL SE

(71) Anmelder: Erich Schumm GmbH
Erich-Schumm-Strasse 2-4
D-7157 Murrhardt(DE)

(72) Erfinder: Frank, Ernst
Wiesenweg 3
D-7150 Backnang(DE)

(74) Vertreter: Dreiss, Uwe, Dr. jur. Dipl.-Ing. M.Sc. et al,
Patentanwälte Dreiss, Hosenthien & Fuhlendorf
Gerokstrasse 6
D-7000 Stuttgart 1(DE)

(54) Eierprüfgerät.

(57) Es ist ein Eierprüfgerät beschrieben, das mit einer trichterförmigen, ein Ende eines Eies (7) aufnehmenden Eiaufnahme (4), einer dazu konzentrisch am unteren Trichterende vorgesehenen Lichtdurchlaßöffnung (5) und einer etwa in der Längsachse (9) der Eiaufnahme (4) angeordneten, über mindestens einen Schaltkontakt (18, 18') schaltbaren elektrischen, vorzugsweise batteriegespeisten Lichtquelle (6) versehen ist.

Damit das Eierprüfgerät bei einfachem Aufbau sehr standfest ist und einen geringen Stromverbrauch aufweist, ist die trichterförmige Eiaufnahme (4) gegen eine Feder (31) verschiebbar auf einem Bodenteil (1) in Richtung der Längsachse (9) verschiebbar geführt. Dabei sind zwischen der Eiaufnahme (4) und dem Bodenteil (1) die Schaltkontakte (18, 18') vorgesehen und durch Niederdrücken der Eiaufnahme (4) schaltbar.

Fig.1

## Beschreibung

Die Erfindung betrifft ein Eierprüfgerät mit einer trichterförmigen, ein Ende eines Eies aufnehmenden Eiaufnahme, einer dazu konzentrisch am unteren Trichterende vorgesehenen Lichtdurchlaßöffnung und einer etwa in der Längsachse der Eiaufnahme angeordneten über mindestens einen Schaltkontakt schaltbaren elektrischen, vorzugsweise batteriegespeisten Lichtquelle.

Bei einem solchen aus dem DE-GM 19 39 386 bekannten Eierprüfgerät kann auf die Eiaufnahme eine becherförmige Abdeckung aufgebracht werden, in der eine Glühlampe und eine Batterie und ein Schaltkontakt angeordnet ist, der beim Aufsetzen der Abdeckung auf die Eiaufnahme geschlossen wird und so die Glühlampe aufleuchtet. Unterhalb des Eies ist in der Eiaufnahme ein Spiegel angeordnet, der über ein Sichtfenster eine Eikontrolle gestattet. Nachteilig ist, daß die Abdeckung mit der eingebauten Batterie relativ schwer ist und hoch baut. Es besteht damit die Gefahr des Umkippens. Weiter brennt bei aufgesetzter Abdeckung immer die Glühlampe, so daß das Gerät nicht platzsparend zur Aufbewahrung zusammengesteckt werden kann. Verbunden hiermit ist ein relativ hoher Batterieverbrauch. Zur sorgfältigen Eikontrolle kann während des Prüfens das Ei nicht gedreht werden, da es durch die Abdeckung vollständig verdeckt ist. Der Spiegel ist Verschmutzungen und Beschädigungen ausgesetzt.

Aufgabe der vorliegenden Erfindung ist es in Vermeidung der geschilderten Nachteile, ein Eierprüfgerät der eingangs genannten Art zu schaffen, das bei einfachem Aufbau sehr standfest ist und einen geringen Stromverbrauch aufweist.

Zur Lösung dieser Aufgabe sieht die Erfindung vor, daß die trichterförmige Eiaufnahme gegen eine Feder verschiebbar auf einem Bodenteil in Richtung der Längsachse verschiebbar geführt ist, daß zwischen der Eiaufnahme und dem Bodenteil der bzw. die Schaltkontakte vorgesehen und durch Niederdrücken der Eiaufnahme schließbar sind.

Hierdurch kann in einfacher Weise während der Prüfung die Lichtquelle durch Niederdrücken der Eiaufnahme eingeschaltet werden. Beim Loslassen wird durch die Feder der Schaltkontakt unterbrochen, so daß keine unbeabsichtigte Stromkreisschließung erfolgen kann. Während der Kontrolle ist das Ei frei zugänglich und kann gedreht werden, wobei allerdings zur Verbesserung der Kontrolle ein Sichthut über das Ei aufgesetzt werden kann, wenn eine Kontrolle bei besonders hellem Umgebungslicht durchgeführt werden soll.

Vorteilhaft kann der Verschiebeweg der Eiaufnahme in Richtung der Federkraft durch mindestens einen Anschlag und in Richtung gegen die Federkraft durch den bzw. die Schaltkontakte begrenzt sein. Besonders vorteilhaft kann als Anschlag ein Absatz und mindestens ein mit diesem zusammenwirkender Vorsprung dienen, wobei der Absatz am Bodenteil und der bzw. die Vorsprünge an der Eiaufnahme oder umgekehrt angeordnet sein können.

Herstellungsmäßig besonders einfach und sehr formstabil können der Bodenteil und die Eiaufnahme

4003 280

einander angepaßte zylindrische Führungsflächen aufweisen und es kann weiter der Absatz durch einen Durchmessersprung gebildet sein. Weiter können als Vorsprünge am Umfang verteilt mindestens zwei gegen den Absatz gerichtete Nasen vorgesehen sein, die die den Absatz aufweisende zylindrische Führungsfläche bei der Montage durch die Eigenelastizität der die Nasen tragenden Führungsfläche federnd übergleiten können. Zweckmäßigerweise sind am Umfang verteilt drei oder vier Nasen vorgesehen, die auch noch ohne größere Kraftaufwendungen eine leichte Demontage von Bodenteil und Eiaufnahme erlauben, ohne daß Materialbeschädigungen oder Überbeanspruchungen auftreten können.

Eine besonders hohe Standfestigkeit des Prüfgeräts kann dadurch erreicht werden, daß der Bodenteil topfartig ausgebildet ist und seine Außenseite als Führungsfläche für die Eiaufnahme dient. Weiter können zur Verlagerung des Schwerpunkts nach unten mindestens ein vorzugsweise aber einander gegenüberliegend zwei zur Längsachse parallele Aufnahmefächer für jeweils eine Batterie vorgesehen sein, wobei die Batterien durch die Eigenelastizität der entsprechend vorgeformten Aufnahmefächer sicher aber leicht austauschbar gehalten sind.

Besonders einfach montierbar und ohne zusätzliche Verbindungsteile kann am Boden des topfartigen Bodenteils eine Glühlampenfassung mit einer Glühlampe vorgesehen sein, die über eine Verbindungsleitung mit einem unten angeordneten Pol der Batterie verbunden ist. Zweckmäßigerweise sind etwa symmetrisch zwei Verbindungsleitungen vorgesehen, die jeweils einen Anschluß der Glühlampenfassung mit einem der unten angeordneten Pole der Batterien verbindet.

Gewichtsparend können die Verbindungsleitungen die Lampenfassung tragende Metallstege sein, die am Boden des Bodenteils zwischen Vorsprüngen gehalten sind, wobei diese Metallstege parallel zum Boden unten an den Aufnahmefächern für die Batterien durch Schlitze in diese hineinragen.

Ohne besondere Befestigungsvorkehrungen kann die Lampenfassung zwischen diesen Vorsprüngen aufgenommen sein. Zweckmäßigerweise sind mindestens vier zu den Metallstegen parallele rippenartige Vorsprünge vorgesehen, von denen zwei diagonal einander gegenüberliegende Vorsprünge etwa der Höhe der Schlitze der Aufnahmefächer entsprechen und jeweils auf der Seite der Schlitze vorgesehen sind und es können die anderen Vorsprünge zur Abstützung der Glühlampenfassung höher ausgebildet sein.

Besonders einfach können die Metallstege mit der Lampenfassung einstückig durch Drehen federnd über die niedrigen Vorsprünge eindreh- und einschnappbar sein, wobei dann die Schlitze am unteren Ende der Aufnahmefächer auch von einer Seite her bis etwa über die Mitte der Aufnahmefächer sich diagonal gegenüberliegend erstrecken.

Ein besonders geringes Konstruktionsgewicht bei einfacher Montage und leichter Reinigbarkeit des Geräts kann dadurch erreicht werden, daß die Glühlampenfassung und die Verbindungsleitungen zu den Batteriepolen als Baueinheit einstückig ausgebildet ist und daß die Aufnahmefächer bis zum Boden reichende zur Längsachse parallele und zur Mitte hin offene Schlitze aufweisen, durch die die Baueinheit von oben einlegbar und durch die Batterien gehalten ist.

Eine gedrungene platzsparende Ausbildung des Geräts kann dadurch erreicht werden, daß die trichterförmige Eiaufnahme einen zylindrischen Wandteil aufweist, der die mit dem Bodenteil zusammenwirkende Führungsfläche abgibt und weiter einen senkrecht zur Längsachse verlaufenden Wandteil, wobei dann an diesem Wandteil mindestens einer der Schaltkontakte vorgesehen ist, zweckmäßigerweise als elektrisch leitender Kontaktring.

Weitere erfindungsgemäße Ausbildungen sind den Unteransprüchen zu entnehmen und werden mit ihren Vorteilen in der nachstehenden Beschreibung näher erläutert. In den beigefügten Zeichnungen zeigt:

Figur 1          einen Schnitt durch ein Eierprüfgerät und

Figur 2          eine Draufsicht auf den Bodenteil
                 des in Fig. 1 dargestellten Eierprüfgeräts ohne Batterien.

Beim dargestellten Ausführungsbeispiel eines Eierprüfgeräts ist auf einem topfförmigen Bodenteil 1 auf
dessen zylindrischem Wandteil 2, dessen Außenfläche eine Führungsfläche 3 abgibt, eine trichterförmige Eiaufnahme 4 aufgesetzt. Diese Eiaufnahme 4 weist an ihrem inneren unteren Ende eine Lichtdurchlaßöffnung 5 auf, durch die das Licht einer Lichtquelle 6 in Form einer Glühlampe nach oben austreten kann zum Durchleuchten eines zum Prüfen in die Eiaufnahme 4 eingelegten Eies 7.

Die Eiaufnahme 4 weist an ihrem oberen Ende eine konzentrische Erhöhung 8 auf, die in einen senkrecht zur Längsachse 9 verlaufenden Wandteil 10 übergeht. Die Erhöhung 8 dient zum zentrierten Aufsetzen einer napfförmigen Sichtabdeckung 11, die an ihrem oberen Ende eine Durchblicköffnung 12 trägt zum Kontrollieren des Eies 7 bei ungünstigen, besonders hellen Lichtverhältnissen. Der Wandteil 10 geht in einen zylindrischen Wandteil 13 über, dessen zylindrische Innenfläche eine Führungsfläche 14 abgibt,die auf der Führungsfläche 3 des Bodenteils 1 gleiten kann.

Der Bodenteil 1 weist an seinem unteren Ende einen Absatz 15 auf, der als Anschlag 16 für am unteren Ende der Eiaufnahme 4 angeordneten Vorsprünge 17 in Form von Nasen dient. Am Umfang verteilt sind insgesamt beim Ausführungsbeispiel vier solche nasenförmige Vorsprünge 17 angeordnet, die durch elastische Umfangsverformung bei der Montage leicht über die Führungsfläche 3 des Bodenteils 1 übergeschoben werden können und zur Demontage auch wieder über den Absatz 15 bei einer gewissen Kraftanwendung, allerdings ohne Materialbeschädigung zurückgeschoben werden können.

Auf der Unterseite des Wandteils 10 ist zwei Schaltkontakte 18,18' mit zwei Batterien 19 19' bildend ein Kontaktring 20 angeordnet. Besonders einfach ist der Kontaktring 20 ein eingeklebter elektrisch leitender Folienring, beispielsweise aus Aluminiumfolie.

Die beiden Batterien 19,19' sind in zur Längsachse 9 parallele Aufnahmefächer 21 bzw. 21' des Bodenteils 1 eingeschoben und durch eine elastische Vorspannung der Wände der Aufnahmefächer 21,21' kraftschlüssig, aber leicht austauschbar gehalten.

In der Mitte auf der Oberseite des Bodens 22 des Bodenteils 1 sind rippenartige Vorsprünge 23,24 und 25 und 23', 24' und 25' angeordnet und zwischen diesen ist eine Baueinheit 26 gehalten, die aus einer Lampenfassung 27, der Lichtquelle 6 und zwei einander gegenüberliegende Metallstege 28 und 28' besteht. Die Vorsprünge 23 und 23' dienen zur senkrechten Lagehaltung der Lampenfassung 27. Die Vorsprünge 25 und 25' und die Vorsprünge 23 und 23' sind höher ausgebildet als die diagonal zueinander angeordneten Vorsprünge 24 und 24'. Ferner sind parallel und in Höhe des Bodens 22 unten in den Aufnahmefächern 21 bis über die Mitte dieser Fächer gehende Schlitze 29 bzw. 29' angeordnet. Durch diese seitlich offenstehenden Schlitze 29,29' können die Enden der Metallstege 28,28' durch eine Drehbewegung eingeschoben werden, wobei gleichzeitig diese Metallstege 28,28' unter Anhebung der Lampenfassung 27 über die Vorsprünge 24 und 24' hinweggeschwenkt werden können. Durch die Eigenelastizität wird dann zusammen mit den Metallstegen 28,28' die Lampenfassung 27 nach unten zwischen die Vorsprünge 23,23' gedrückt und dort gehalten. Durch die Vorsprünge 23 bis 25 wird ein unbeabsichtigtes Lösen der Baueinheit 26 verhindert.

Der Bodenteil 1 wird aus Kunststoff durch Spritzen hergestellt. Um die Schlitze 29,29' ohne Schieber herstellen zu können, sind im Boden 22 des Bodenteils 1 Durchbrüche 30,30' für entsprechende Werkzeugvorsprünge vorgesehen.

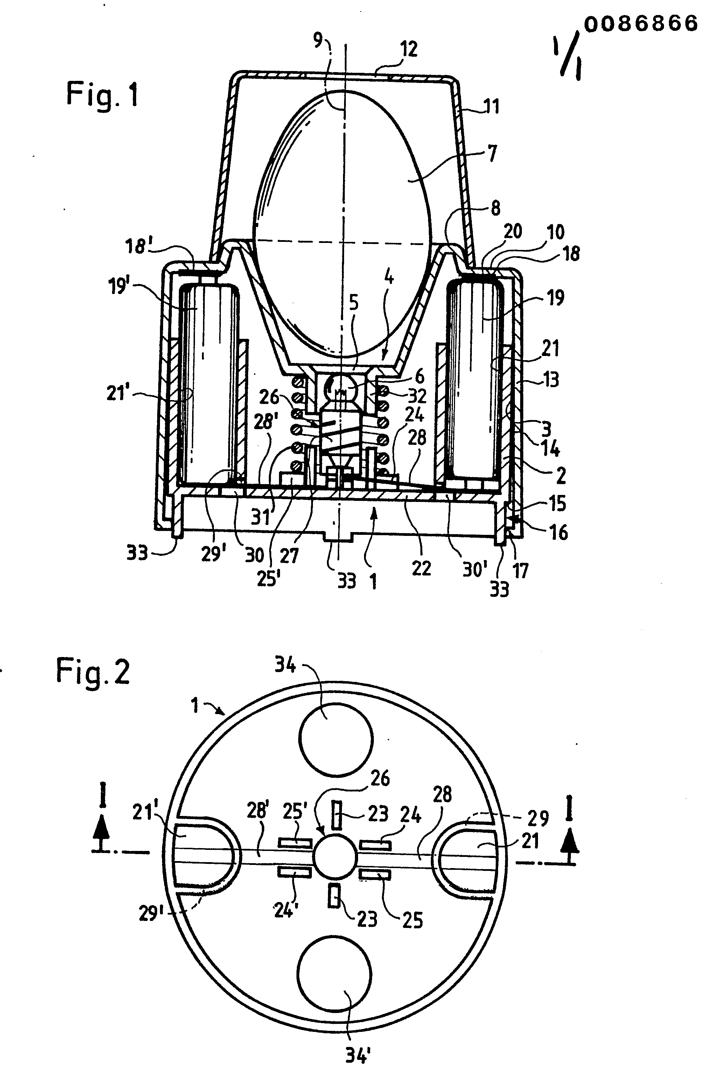

Wie aus Figur 1 ersichtlich ist der Metallsteg 28' mit dem zentrischen Mittelteil der Lampenfassung 27 und der Metallsteg 28 mit der metallischen Einschraubfassung elektrisch leitend verbunden, so kann beim Niederdrücken der Eiaufnahme 4 gegen die Wirkung einer Feder 31 der Stromkreis über die Schaltkontakte 18,18' geschlossen und die Lichtquelle 6 zum Aufleuchten gebracht werden. Die als Druckfeder ausgebildete Feder 31 ist auf einem zylindrischen Verlängerungsteil 32 der Eiaufnahme 4 gehalten und stützt sich auf den etwas höher ausgebildeten Vorsprüngen 23,23', 25,25' ab. Die Federkraft ist so bemessen, daß durch die Feder die Eiaufnahme 4 zusammen mit dem Ei 7 und auch dem aufgesetzten Sichthut 11 sicher angehoben, aber durch ein leichtes Niederdrücken mit der Hand gegen den Bodenteil 1 verschoben werden kann um die Lichtquelle 6 zur Eiprüfung einzuschalten. Der Bodenteil 1 weist zum Aufstellen des Prüfgeräts eine Aufstellfläche 33 auf, die durch beispielsweise vier am Umfang verteilte Füße gebildet sein kann.

- 12 -

Zur Gewichtseinsparung und leichten Reinigbarkeit und auch zur Erleichterung der Montage und Demontage können im Boden 22 noch zwei oder auch mehr Durchbrüche 34,34' vorgesehen sein.

- Ende der Beschreibung -

4003 280

# DREISS, HOSENTHIEN & FUHLENDORF

**0086866**

| HANS LANGOSCH<br>Dipl.-Ing. (1963 - 1981)<br>UWE DREISS<br>Dr. Jur., Dipl.-Ing., M. Sc.<br>HEINZ HOSENTHIEN<br>Dr.-Ing., Dipl.-Ing.<br>JÖRN FUHLENDORF<br>Dipl.-Ing. | **PATENTANWÄLTE**<br>Beim Europäischen Patentamt zugelassene Vertreter<br>European Patent Attorneys | **D-7000 STUTTGART 1**<br>GEROKSTRASSE 6<br>TF (07 11) 24 57 34/44<br>TG IDEAPAT<br>TX 7-22 247 Idea d<br>P für Besucher |

DREISS, HOSENTHIEN & FUHLENDORF, D-7000 STUTTGART 1

Anmelder:

Erich Schumm GmbH

Erich-Schumm-Straße 2 - 4

D-7157  Murrhardt

| Amtl. Akt. Z.<br>Off. Ser. No. | Ihr Zeichen<br>Your Ref. | Unser Zeichen<br>Our Ref.<br>4003 280 | Datum<br>Date<br>8.6.1982 F/Sf |
|---|---|---|---|

Titel: Eierprüfgerät

## Patentansprüche

1. Eierprüfgerät mit einer trichterförmigen, ein
Ende eines Eies (7) aufnehmenden Eiaufnahme (4),
einer dazu konzentrisch am unteren Trichterende vorgesehenen Lichtdurchlaßöffnung (5),
einer etwa in der Längsachse (9) der Eiaufnahme
(4) angeordneten, über mindestens einen Schaltkontakt (18, 18') schaltbaren elektrischen, vorzugsweise batteriegespeisten Lichtquelle (6),
<u>dadurch gekennzeichnet</u>, daß die trichterförmige
Eiaufnahme (4) gegen eine Feder (31) verschiebbar auf einem Bodenteil (1) in Richtung der
Längsachse (9) verschiebbar geführt ist, daß zwischen der Eiaufnahme (4) und dem Bodenteil (1) der bzw. die Schaltkontakte (18, 18') vorgesehen und durch Niederdrücken der Eiaufnahme
(4) schaltbar sind.

2. Eierprüfgerät nach Anspruch 1, <u>dadurch gekenn-
zeichnet</u>, daß der Verschiebeweg der Eiaufnahme
(4) in Richtung der Federkraft durch mindestens einen Anschlag (16) und in Richtung gegen
die Federkraft durch den bzw. die Schaltkontakte
(18, 18') begrenzt ist.

3. Eierprüfgerät nach Anspruch 2, <u>dadurch gekenn-
zeichnet</u>, daß als Anschlag (16) ein Absatz (15)
und ein mit diesem zusammenwirkender Vorsprung
(17) dienen, wobei der Absatz (15) am Bodenteil
(1) und der bzw. die Vorsprünge (17) an der
Eiaufnahme (4) oder umgekehrt angeordnet sind.

4. Eierprüfgerät nach Anspruch 3, <u>dadurch gekenn-
zeichnet</u>, daß der Bodenteil (1) und die Eiaufnahme (4) aneinander angepasste zylindrische
Führungsflächen (3, 14) aufweisen und der Absatz
(15) durch einen Durchmessersprung gebildet ist
und daß als Vorsprung (17) am Umfang verteilt
mindestens zwei gegen den Absatz (15) gerichtete Nasen vorgesehen sind, die die den Absatz
(15) aufweisende zylindrische Führungsfläche
(3) bei der Montage durch die Eigenelastizität der die Nasen tragenden Führungsfläche (14) federnd übergleiten können.

5. Eierprüfgerät nach Anspruch 3 oder 4,
dadurch gekennzeichnet, daß der Absatz (15)
am unteren Ende des Bodenteils (1) vorgesehen ist und daß der untere Rand des Bodenteils (1) als Aufstellfläche (33) des Gerätes dient.

6. Eierprüfgerät nach einem der Ansprüche 1 bis
5, dadurch gekennzeichnet, daß der Bodenteil
(1) topfartig ausgebildet und seine Außenseite
als Führungsfläche (3) für die Eiaufnahme (4)
dient und daß mindestens ein vorzugsweise aber
einander gegenüberliegend zwei zur Längsachse
(9) parallele Aufnahmefächer (21, 21') für jeweils eine Batterie (19) bzw. (19') vorgesehen
sind.

7. Eierprüfgerät nach Anspruch 6, <u>dadurch gekenn-
zeichnet</u>, daß am Boden (22) des topfartigen
Bodenteils (1) eine Glühlampenfassung (27) mit
einer Glühlampe vorgesehen ist, die über eine
Verbindungsleitung mit dem unten angeordneten
Pol der Batterie (19) bzw. (19') verbunden ist.

8. Eierprüfgerät nach Anspruch 7, <u>dadurch gekenn-
zeichnet</u>, daß die Verbindungsleitungen die
Lampenfassung tragende Metallstege (28, 28')
sind, die am Boden (22) des Bodenteils (1) zwischen Vorsprüngen (24, 25) gehalten sind und
parallel zum Boden (22) unten in die Aufnahmefächer (21, 21') für die Batterien (19, 19')
durch Schlitze (29, 29') in diese hineinragen,
und daß die Schlitze (29, 29') am unteren Ende
der Aufnahmefächer (21, 21') sich von einer
Seite her bis etwa über die Mitte der Aufnahmefächer (21, 21') diagonal gegenüberliegend erstrecken.

9. Eierprüfgerät nach Anspruch 7 und 8, <u>dadurch
gekennzeichnet</u>, daß die Lampenfassung (27) zwischen den Vorsprüngen (23 bis 25) aufgenommen
ist, daß mindestens vier zu den Metallstegen (28, 28') parallele rippenartige Vorsprünge
(24, 24'; 25, 25') vorgesehen sind, von denen
zwei diagonal einander gegenüberliegende Vorsprünge (24, 24') etwa der Höhe der Schlitze
(29, 29') der Aufnahmefächer (21, 21') entsprechen und jeweils auf der Seite der Schlitze
(29, 29') vorgesehen sind und die beiden anderen Vorsprünge (25, 25') zur Abstützung der
Glühlampenfassung (27) höher ausgebildet sind.

10. Eierprüfgerät nach Anspruch 6 und 7, dadurch
gekennzeichnet, daß die Lampenfassung (27) und
die Verbindungsleitungen zu den Batteriepolen
als Baueinheit (26) ausgebildet ist und daß die
Aufnahmefächer (21, 21') bis zum Boden reichende
zur Längsachse (9) parallele zur Mitte hin gerichtete Schlitze aufweisen.

11. Eierprüfgerät nach einem der Ansprüche 1 bis 10,
dadurch gekennzeichnet, daß die trichterförmige
Eiaufnahme (4) einen zylindrischen Wandteil (13)
aufweist, der die mit dem Bodenteil (1) zusammenwirkende Führungsfläche (14) abgibt und einen
senkrecht zur Längsachse (9) verlaufenden Wandteil (10), daß an diesem Wandteil (10) mindestens einer der Schaltkontakte (18, 18') vorgesehen ist und daß der senkrecht zur Längsachse (9) verlaufende Wandteil (10) eine umlaufende konzentrische Erhöhung (8) für die
zentrierte Halterung eines das Ei abdeckenden
Sichthuts (11), wobei der Sichthut (11) eine
Durchblicköffnung (12) für die Eikontrolle
aufweist.

12. Eierprüfgerät nach Anspruch 11, dadurch gekennzeichnet, daß der Schaltkontakt bzw. die
Schaltkontakte (18, 18') als elektrisch leitender Kontaktring (20) ausgebildet ist.

13. Eierprüfgerät nach einem der Ansprüche 1 bis
12, dadurch gekennzeichnet, daß am unteren
inneren Ende der trichterförmigen Eiaufnahme
(4) ein zylindrischer Verlängerungsteil (32)
für die als Druckfeder ausgebildete Feder (31)
vorgesehen ist, und daß die Glühlampe mindestens in der niedergedrückten Einschaltstellung
der Eiaufnahme (4) in den nach oben gegen das
Ei (7) hin offenen Verlängerungsteil (32)
hineinragt.

4003 280

**0086866**

14. Eierprüfgerät nach Anspruch 11, <u>dadurch
    gekennzeichnet</u>, daß zur Bildung der Schaltkontakte (18, 18') die Eiaufnahme (4) aus
    elektrisch leitendem Material, vorzugsweise
    Kunststoff besteht.

- Ende der Ansprüche -

4003 280

Fig. 1

Fig. 2

0086866

1/1

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | FR-A- 667 040 (A. LINDENLAUB) <br> * Anspruch; Figuren 1, 2 * | 1 | G 01 N 33/08 |
| X | FR-A- 838 710 (SIMPLEX) <br> * Figur 1 * | 1,2 | |
| X | FR-A- 775 019 (A. BISSET) <br> * Figuren 1, 3 * | 1,2 | |
| A | CH-A- 49 772 (SCHIEMANN & CO.) <br> * Anspruch; Figuren 1, 2 * | 11 | |

---

RECHERCHIERTE
SACHGEBIETE (Int. Cl. 3)

G 01 N 33/08

Der vorliegende Recherchenbericht wurde für alle Patentansprüche erstellt.

| Recherchenort <br> BERLIN | Abschlußdatum der Recherche <br> 02-05-1983 | Prüfer <br> SCHWARTZ K |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82